# EUROPEAN PATENT APPLICATION

(11) **EP 2 919 430 A1**
(43) Date of publication of application: **16.09.2015**
(21) Application number: 14197816.3
(22) Date of filing: 12.12.2014
(51) Int. Cl.: H04L 29/06, H04W 12/06, H04W 4/00, A61B 5/024, G06F 21/32, G06F 21/35

(54) **Apparatus and method for authenticating a user using a wearable electronic device**

(30) Priority: 11.03.2014 KR 20140028480
(71) Applicant: Samsung Electronics Co., Ltd, Gyeonggi-do 443-742 (KR)
(72) Inventor: Yoon, Byoung-Uk, 443-742 Gyeonggi-do (KR); Heo, Chang-Ryong, 443-742 Gyeonggi-do (KR)
(74) Representative: Nederlandsch Octrooibureau

(57) **Abstract**

An apparatus and method for authenticating a user in an electronic device (101) are provided. The method for controlling, by an electronic device (101), a wearable electronic device (103) to authenticate a user includes receiving biometric information of the user from the wearable electronic device (103), determining whether the user has been registered in the wearable electronic device (103), based on the biometric information and stored authentication information, and controlling the wearable electronic device (103) to authenticate the user according to a result of the determination.

## Description

### TECHNICAL FIELD

The present disclosure relates to an apparatus and method for authenticating of a user.

### BACKGROUND

Electronic devices, such as smart phones and tablet terminals, provide various useful functions through various applications and have evolved so that they now provide various types of information and advanced functions in addition to a voice call function. For example, an electronic device may be connected to an external device. In that case, the electronic device uses the external device to provide various functions that fail to be provided by the electronic device. For example, based on a number or pattern pre-registered by a user, the electronic device may determine whether the current user is a user pre-registered in the electronic device.

However, since the pre-registered number or pattern may be easily exposed to other users, the security of the electronic device may be easily undermined or disabled.

The above information is presented as background information only to assist with an understanding of the present disclosure. No determination has been made, and no assertion is made, as to whether any of the above might be applicable as prior art with regard to the present disclosure.

### SUMMARY

Aspects of the present disclosure are to address at least the above-mentioned problems and/or disadvantages and to provide at least the advantages described below. Accordingly, an aspect of the present disclosure is to provide an apparatus and method for authenticating a user based on unique biometric information of the user in order to maximize the security of an electronic device.

Another aspect of the present disclosure is to provide an apparatus and method for extracting unique biometric information of a user by using an external device, which is capable of communicating with an electronic device, in order to maximize the security of the electronic device.

In accordance with an aspect of the present disclosure, an electronic device for controlling a wearable electronic device to authenticate a user is provided. The electronic device includes a short-range wireless communication unit configured to receive biometric information of the user from the wearable electronic device, and a control unit configured to determine whether the user has been registered in the wearable electronic device, based on the biometric information and stored authentication information, and control the wearable electronic device to authenticate the user according to a result of the determination.

In accordance with another aspect of the present disclosure, a wearable electronic device for authenticating a user is provided. The wearable electronic device includes an image sensor and a light emitter mounted on one surface of the wearable electronic device, and a lens or a waveguide including at least one opening on a rear surface thereof.

In accordance with another aspect of the present disclosure, a method for controlling, by an electronic device, a wearable electronic device to authenticate a user is provided. The method includes receiving biometric information of the user from the wearable electronic device, determining whether the user has been registered in the wearable electronic device, based on the biometric information and stored authentication information, and controlling the wearable electronic device to authenticate the user according to a result of the determination.

In accordance with another aspect of the present disclosure, a method for authenticating a user by a wearable electronic device is provided. The method includes generating biometric information of the user, generating pulse information of the user, and generating a vein image of the user.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a schematic diagram of a system according to an embodiment of the present disclosure;
FIG. 2 is a perspective view of a wearable electronic device according to an embodiment of the present disclosure;
FIGS. 3A, 3B, and 3C illustrate a sensor unit of a wearable electronic device according to an embodiment of the present disclosure;
FIG. 4 illustrates a coupling unit of a wearable electronic device according to an embodiment of the present disclosure;
FIG. 5 illustrates generating a vein image in a wearable electronic device according to an embodiment of the present disclosure;
FIG. 6A is a flowchart illustrating a process of executing an initial authentication setting mode in a wearable electronic device according to an embodiment of the present disclosure;
FIG. 6B is a flowchart illustrating a process of setting authentication information in a wearable electronic device according to an embodiment of the present disclosure;
FIG. 7 is a flowchart illustrating a process of setting authentication information in a wearable electronic device according to an embodiment of the present disclosure;
FIG. 8 is a flowchart illustrating a process of performing a user authentication operation in a wearable electronic device according to an embodiment of the present disclosure;
FIG. 9 is a flowchart illustrating a process of performing a user authentication operation in a wearable electronic device according to an embodiment of the present disclosure;
FIG. 10 is a flowchart illustrating a process of detecting a coupling of a wearable electronic device and performing a user authentication operation in a wearable electronic device according to an embodiment of the present disclosure;
FIG. 11 is a flowchart illustrating a process of executing an application of a wearable electronic device according to a hand of a user to which the wearable electronic device is coupled according to an embodiment of the present disclosure;
FIG. 12 is a flowchart illustrating a process of performing an application according to a user in a wearable electronic device according to an embodiment of the present disclosure;
FIG. 13 is a block diagram of an electronic device connected with a wearable electronic device according to an embodiment of the present disclosure; and
FIG. 14 is a block diagram of a wearable electronic device according to an embodiment of the present disclosure.

Throughout the drawings, like reference numerals will be understood to refer to like parts, components, and structures.

### DETAILED DESCRIPTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the present disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

By the term "substantially" it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

An electronic device according to an embodiment of the present disclosure may be any portable mobile electronic device, examples of which include video phones, mobile phones, smart phones, International Mobile Telecommunication 2000 (IMT-2000) terminals, Wideband Code Division Multiple Access (WCDMA) terminals, Universal Mobile Telecommunication Service (UMTS) terminals, Personal Digital Assistants (PDAs), Portable Multimedia Players (PMPs), Digital Multimedia Broadcasting (DMB) terminals, e-books, Portable Computers (PCs) (e.g., notebook PCs and tablet PCs), digital cameras, and the like.

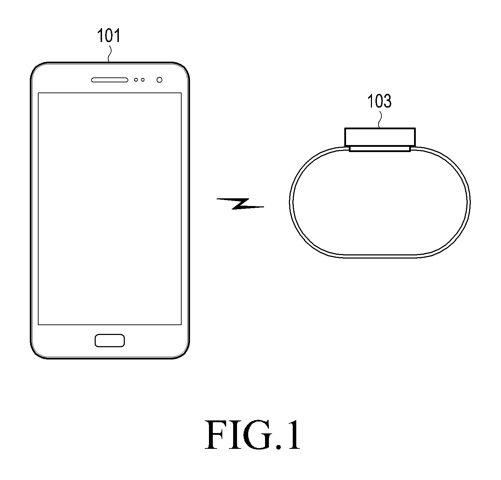

FIG. 1 is a schematic diagram of a system according to an embodiment of the present disclosure.

Referring to FIG. 1, the system may include an electronic device 101 and a wearable electronic device 103.

The electronic device 101 may be a mobile terminal capable of performing data communication and voice and video calls. In an embodiment, the electronic device 101 may have at least one screen that may display execution results of at least one application. The electronic device 101 may include, for example, smart phones, tablet PCs, 3-Dimensional Televisions (3D TVs), smart TVs, Light Emitting Display (LED) TVs, Liquid Crystal Display (LCD) TVs, tablet PCs, and the like and may also include any other device that may communicate with peripheral devices or remote terminals. The electronic device 101 may include wireless Bluetooth communication devices, Near Field Communication (NFC) devices, WiFi Direct communication devices, and wireless Access Points (APs). Also, the electronic device 101 may be wiredly or wirelessly connected to other devices such as a portable terminal, a smart phone, a tablet PC, a desktop PC, an input device, the wearable electronic device 103, a camera, a server, and the like.

When coupled to a wrist of a user, the wearable electronic device 103 may detect biometric information of the user. Herein, the biometric information refers to unique biometric information of the user, and may include, for example, at least one of a sweat component, a vein image, an electrical signal of a pulse, and a skin depth image of the user. Also, the wearable electronic device 103 may process the detected biometric information at the request of the user, and may store the processed biometric information. Also, for example, the wearable electronic device 103 may periodically detect biometric information of the user, and may store the detected biometric information. Also, the wearable electronic device 103 may store basic information of the user. For example, the basic information of the user may include at least one of time, temperature, humidity, and wind conditions, under which the biometric information of the user is measured, a blood pressure of the user, disease information of the user, notes on the user, and the like. Also, through wireless communication, the wearable electronic device 103 may transmit the detected biometric information to at least one of the electronic device 101, a separate server, another wearable electronic device, and the like, and may receive the basic information of the user from at least one of the electronic device 101, a separate server, another wearable electronic device, and the like.

According to an embodiment of the present disclosure, when coupled to a wrist of the user, the wearable electronic device 103 may detect biometric information of the user. The wearable electronic device 103 may compare the detected biometric information with prestored authentication information and deactivate a user authentication mode. Herein, the user authentication mode is a mode for authenticating the user, and refers to a mode for determining whether the current user of the wearable electronic device 103 has been registered in the wearable electronic device 103. For example, the prestored authentication information may include biometric information of the user registered in the wearable electronic device 103. Herein, the biometric information may include, for example, at least one of a sweat component, a vein image, an electrical signal of a pulse, a skin depth image of the user, and the like. In other words, the wearable electronic device 103 may independently detect the biometric information of the user and determine whether to deactivate the user authentication mode. Also, for example, the prestored authentication information refers to information, which is detected when the user first wears the wearable electronic device 103, and may include at least one of an average heart rate of the user, a skin color image of the user, a vein image of the user, and the like.

In the above embodiment, the system includes the electronic device 101 and the wearable electronic device 103. However, in another embodiment, the system may include the wearable electronic device 103 and a separate server, and the server may perform the same function as the electronic device 101.

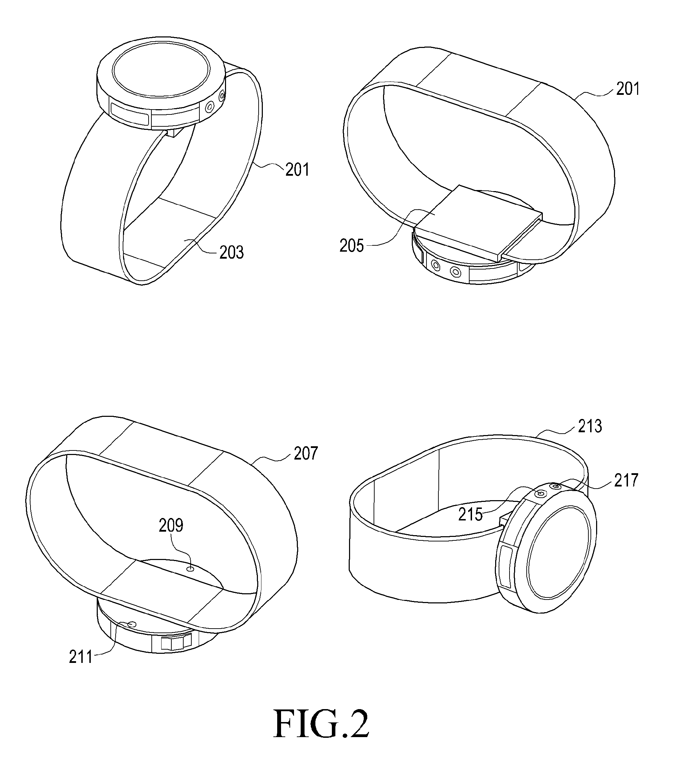

FIG. 2 is a perspective view of a wearable electronic device according to an embodiment of the present disclosure.

Referring to FIG. 2, the wearable electronic device may include an image sensor and a light emitter, and may generate a vein image of a wrist or a back of a hand of the user by using the image sensor and the light emitter. Also, the wearable electronic device may transmit the generated vein image to the electronic device.

The image sensor and the light emitter body may be mounted on the wearable electronic device in the following ways.

In a first way, an image sensor 205 and a light emitter 203 may be mounted on an inner surface of a wearable electronic device 201. For example, the image sensor 205 and the light emitter 203 may be mounted on the inner surface of the wearable electronic device 201 to face each other. The image sensor 205 may include a sensor for generating a vein image of the wrist of the user, and may be, for example, an infrared camera or a light sensor. The light emitter 203 may include a light-emitting device, and may be, for example, an infrared light-emitting device.

In a second way, an image sensor 209 and a light emitter 211 may be mounted on both edges of a rear surface of a wearable electronic device 207. For example, the image sensor 209 and the light emitter 211 may be mounted on both edges of the rear surface of the wearable electronic device 207 to face each other. The image sensor 209 may include a sensor for generating a vein image of the wrist of the user, and may be, for example, an infrared camera or a light sensor. The light emitter 211 may include a light-emitting device, and may be, for example, an infrared light-emitting device.

In a third way, an image sensor 217 and a light emitter 215 may be mounted on a side surface of a wearable electronic device 213. For example, the image sensor 217 and the light emitter 215 may be mounted on one of the side surfaces of the wearable electronic device 213 to generate a vein image of the back of the hand of the user. The image sensor 217 may include a sensor for generating a vein image of the back of the hand of the user, and may be, for example, an infrared camera or a light sensor. The light emitter 215 may include a light-emitting device, and may be, for example, an infrared light-emitting device.

In a fourth way, an image sensor and a light emitter may be mounted on a rear surface of the wearable electronic device 213. For example, the image sensor and the light emitter may be mounted side by side in a region of the rear surface of the wearable electronic device 213 to generate a vein image of the wrist of the user. Herein, the image sensor may include a sensor for generating a vein image of the wrist of the user, and may be, for example, an infrared camera or a light sensor. The light emitter may include a light-emitting device, and may be, for example, an infrared light-emitting device.

The image sensor and the light emitter, which are included in the wearable electronic device to generate a vein image of the wrist or the back of the hand of the user, may be mounted on the wearable electronic device in various ways other than the above four ways, depending on the shapes of the wearable electronic device.

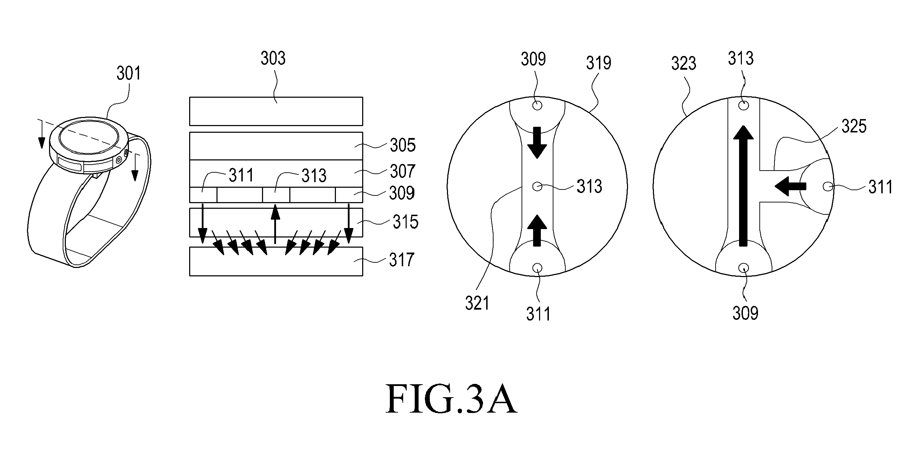

FIGS. 3A to 3C illustrate a sensor unit of a wearable electronic device according to an embodiment of the present disclosure.

The wearable electronic device may perform a user authentication operation by using, for example, a vein image of the user. In that case, the quality of the vein image has to be higher than a reference value in order to perform the user authentication operation. In order to increase the quality of the vein image, light emitted from the light emitter has to be concentrated.

To this end, in an embodiment of the present disclosure, the emitted light may be concentrated in the following way.

Referring to FIG. 3A, an image sensor and a light emitter may be mounted on a rear surface of a wearable electronic device 301. In this case, the wearable electronic device 301 may include a display unit 303, a control unit 305, a memory unit 307, and a light emitter. The light emitter may include a plurality of light-emitting units 309 and 311 emitting light and at least one light-receiving unit 313 receiving the emitted light. Also, the wearable electronic device 301 may include a waveguide 315 for concentrating light into a region of a skin of the user so that the light-receiving unit 313 may sufficiently receive an image of light reflected from a skin 317 of the user.

Herein, the waveguide 315 may have different paths depending on the positions of at least one light-receiving unit 313 and a plurality of light-emitting units 309 and 311 disposed on the rear surface of the wearable electronic device 301. For example, the light-emitting units 309 and 311 may be disposed at both end portions of a rear surface 319 of the wearable electronic device 301 to face each other, and the light-receiving unit 313 may be disposed at an end portion of the rear surface 319 that is perpendicular to the light-emitting units 309 and 311. In this case, the rear surface 319 of the wearable electronic device 301 may include a waveguide 321 for concentrating light into a region of the skin of the user so that the light-receiving unit 313 may sufficiently receive an image of light reflected from the skin 317 of the user.

As another example, the light-emitting units 309 and 311 may be disposed at both end portions of a rear surface 323 of the wearable electronic device 301 to be perpendicular to each other, and the light-receiving unit 313 may be disposed at an end portion of the rear surface 323 to face the light-emitting unit 309. In this case, the rear surface 323 of the wearable electronic device 301 may include a waveguide 325 for concentrating light into a region of the skin of the user so that the light-receiving unit 313 may sufficiently receive an image of light reflected from the skin 317 of the user.

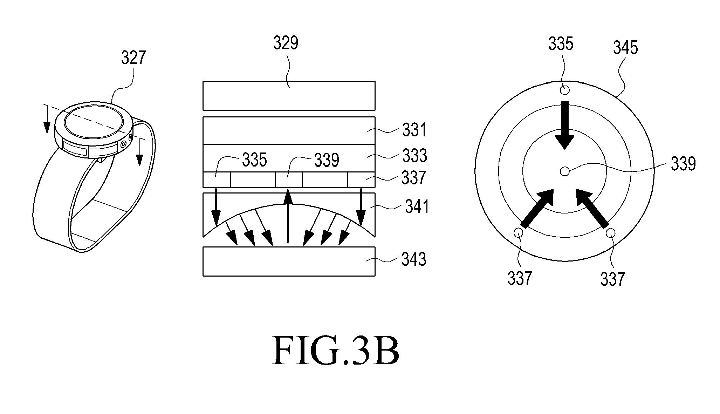

Referring to FIG. 3B, an image sensor and a light emitter may be mounted on a rear surface of a wearable electronic device 327. In this case, the wearable electronic device 327 may include a display unit 329, a control unit 331, a memory unit 333, and a light emitter. The light emitter may include a plurality of light-emitting units 335 and 337 emitting light and at least one light-receiving unit 339 receiving the emitted light. Also, the wearable electronic device 327 may include a lens 341 for concentrating light into a region of a skin of the user so that the light-receiving unit 339 may sufficiently receive an image of light reflected from a skin 343 of the user.

For example, when the light-emitting units 335 and 337 are disposed at an end portion of a rear surface 345 of the wearable electronic device 327 and the light-receiving unit 339 is disposed at a center portion of the rear surface 345, the wearable electronic device 327 may include a lens for concentrating light into a region of the skin of the user so that the light-receiving unit 339 may sufficiently receive an image of light reflected from the skin 343 of the user.

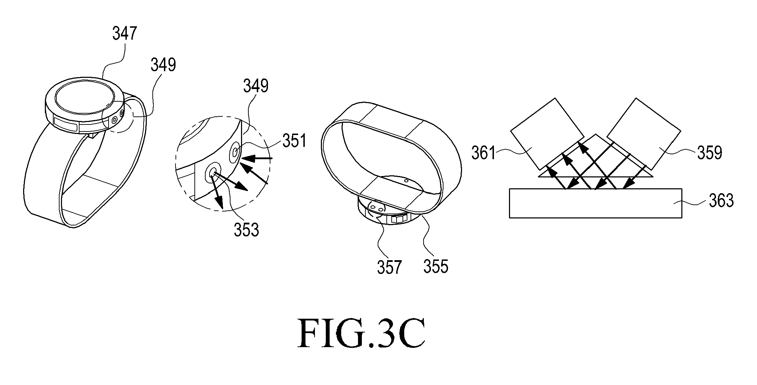

Referring to FIG. 3C, for example, a light emitter may be mounted on a side surface 349 of a wearable electronic device 347. In this case, in the wearable electronic device 347, a light-emitting unit 359 and a light-receiving unit 361 may be disposed such that light emitted by a light-emitting unit 353 may be reflected by the back of the hand of the user and enter at least one light-receiving unit 351. Accordingly, for example, the light-emitting unit 359 and the light-receiving unit 361 may be disposed side by side while being spaced apart from each other by a distance.

Also, for example, a light emitter may be mounted on a rear surface 357 of a wearable electronic device 355. In this case, in the wearable electronic device 355, a light-emitting unit 359 and a light-receiving unit 361 may be disposed at an obtuse or acute angle with each other such that light emitted by at least one light-emitting unit 359 may be reflected by a skin 363 of the user and enter at least one light-receiving unit 361.

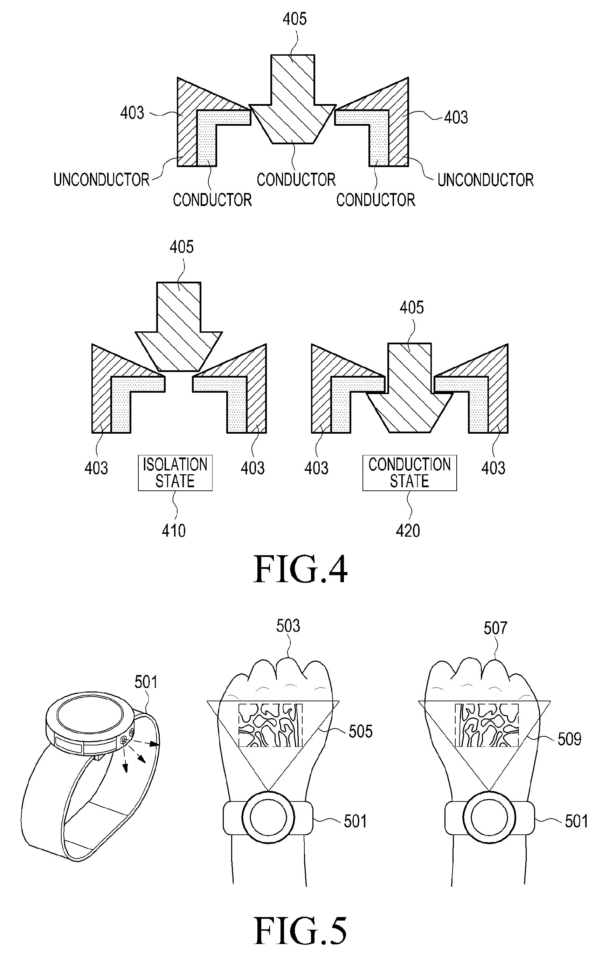

FIG. 4 illustrates a coupling unit of a wearable electronic device according to an embodiment of the present disclosure.

Referring to FIG. 4, a wearable electronic device is provided with a coupling unit including a coupling protrusion 405 and a coupling hook 403. For example, the coupling protrusion 405 may include a conductor, and the coupling hook 403 may include a conductor and a nonconductor. When a voltage is applied to the coupling unit and a current flows between the coupling protrusion 405 and the coupling hook 403, the wearable electronic device may determine that the coupling protrusion 405 and the coupling hook 403 are in a conduction state (420). On the other hand, when a voltage is applied to the coupling unit but no current flows between the coupling protrusion 405 and the coupling hook 403, the wearable electronic device may determine that the coupling protrusion 405 and the coupling hook 403 are in an isolation state (410). For example, when the conductor of the coupling protrusion 405 and the conductor of the coupling hook 403 contact each other, the wearable electronic device may determine that the coupling protrusion 405 and the coupling hook 403 are in a conduction state; and when the conductor of the coupling protrusion 405 and the nonconductor of the coupling hook 403 contact each other, the wearable electronic device may determine that the coupling protrusion 405 and the coupling hook 403 are in an isolation state. When determining that the coupling protrusion 405 and the coupling hook 403 are in a conduction state, the wearable electronic device may determine whether to execute an initial authentication setting mode, or may execute a user authentication mode.

FIG. 5 illustrates generating a vein image in a wearable electronic device according to an embodiment of the present disclosure.

Referring to FIG. 5, a sensor unit for detecting a vein image of the back of the hand of the user may be provided on a side surface of a wearable electronic device 501. For example, when the wearable electronic device 501 is worn around the left wrist of the user, the wearable electronic device 501 may generate a vein image 505 of the left hand 503 of the user. As another example, when the wearable electronic device 501 is worn around the right wrist of the user, the wearable electronic device 501 may generate a vein image 509 of the right hand 507 of the user.

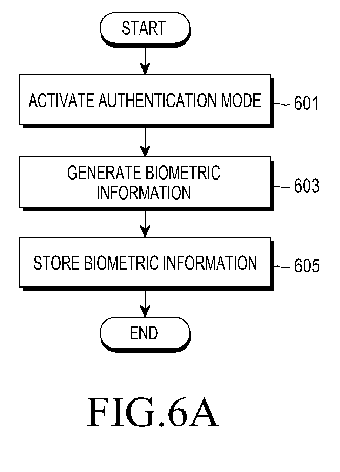

FIG. 6A is a flowchart illustrating a process of executing an initial authentication setting mode in the wearable electronic device according to an embodiment of the present disclosure.

Referring to FIG. 6A, in operation 601, the wearable electronic device may activate an authentication mode. For example, the authentication mode may be a mode for setting authentication information of the user to authenticate the user. Herein, the authentication information may include biometric information of the user. Also, in operation 601, the wearable electronic device may determine the number of users that will use the wearable electronic device.

In operation 603, the wearable electronic device may generate biometric information of the user. The wearable electronic device may use the biometric information to determine a criterion for determining the user. For example, the biometric information may include at least one of a sweat component, a vein image, an electrical signal of a pulse, and a skin depth image of the user.

In operation 605, the wearable electronic device may store the biometric information generated in operation 603. Thereafter, the wearable electronic device may determine the user of the wearable electronic device, generate new authentication information by using the stored biometric information, and store the generated authentication information.

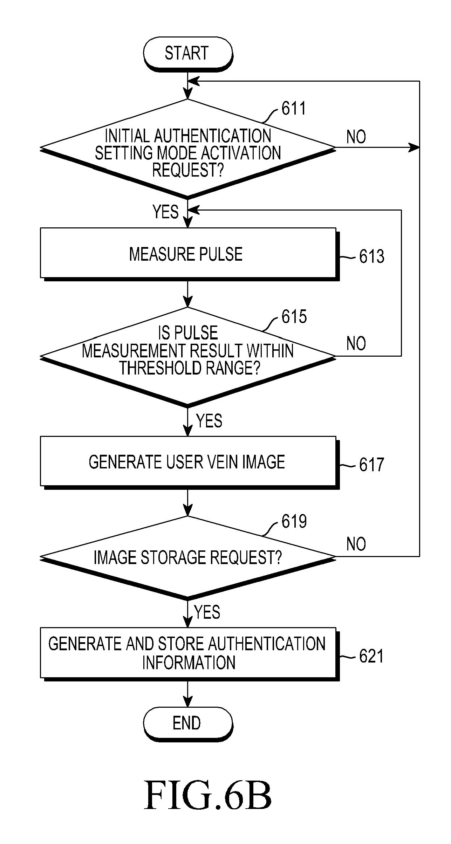

FIG. 6B is a flowchart illustrating a process of setting authentication information in a wearable electronic device according to an embodiment of the present disclosure.

Referring to FIG. 6B, in operation 611, the wearable electronic device may determine whether an initial authentication setting mode activation request is received from the user. In an initial authentication setting mode, unique authentication information of the user may be set to authenticate the user. Initial authentication information may include biometric information of the user registered in the wearable electronic device, and may include, for example, at least one of a sweat component, a vein image, an electrical signal of a pulse, and a skin depth image of the registered user. In other words, whether to execute the initial authentication setting mode of FIG. 6A may be determined in operation 611.

Hereinafter, it is assumed that the wearable electronic device has set and stored the initial authentication information of the user. In operation 613, the wearable electronic device may measure a pulse of the user. For example, in operation 613, the wearable electronic device may measure a pulse of the user for a predtermined. The wearable electronic device may measure a pulse of the user by using a pulse measuring unit included in the sensor unit of the wearable electronic device, and transmit pulse information including a pulse measurement result to the electronic device through short-range wireless communication. Herein, the pulse information may include an electrical signal of the pulse of the user.

In operation 615, the wearable electronic device may determine whether the pulse measurement result is within a threshold range. For example, the threshold range may be determined corresponding to the initial authentication information set through the process of FIG. 6A. When the pulse measurement result is within the threshold range, the wearable electronic device may proceed to operation 617; and when the pulse measurement result is not within the threshold range, the wearable electronic device may return to operation 613.

In operation 617, the wearable electronic device may generate a vein image of the user. Also, the electronic device may transmit a vein image generation request signal for requesting generation of a vein image of the user to the wearable electronic device through short-range wireless communication. The wearable electronic device may generate a vein image of the user by using the image sensor and the light emitter included in the sensor unit of the wearable electronic device, and may transmit the generated vein image to the electronic device through short-range wireless communication.

In operation 619, the wearable electronic device may determine whether a vein image storage request is received from the user. When a vein image storage request is received from the user, the wearable electronic device may proceed to operation 621; and when a vein image storage request is not received from the user, the wearable electronic device may return to operation 611.

In operation 621, the wearable electronic device may generate authentication information including the vein image and the pulse information and store the generated authentication information.

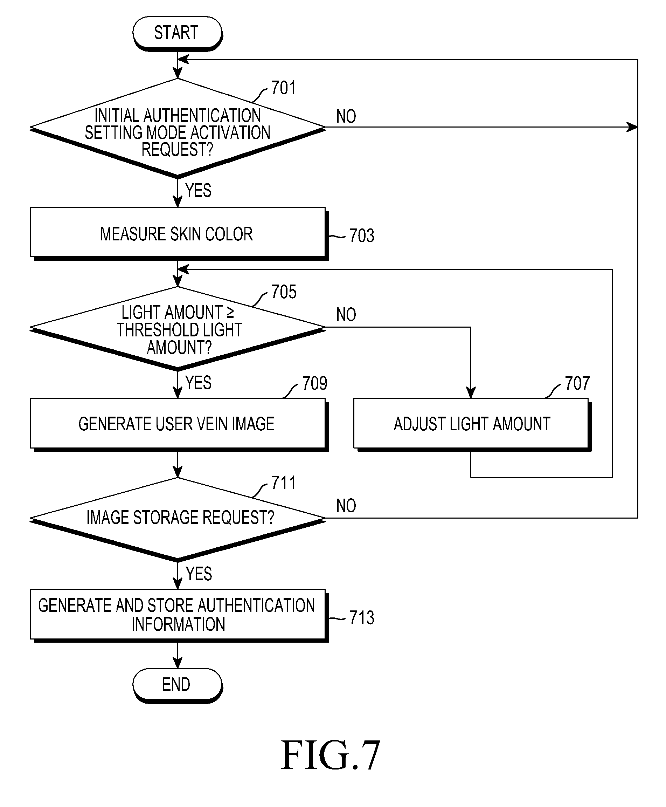

FIG. 7 is a flowchart illustrating a process of setting authentication information in a wearable electronic device according to an embodiment of the present disclosure.

Referring to FIG. 7, in operation 701, the wearable electronic device may determine whether an initial authentication setting mode activation request is received from the user. In an initial authentication setting mode, unique authentication information of the user may be set to authenticate the user. Initial authentication information may include biometric information of the user registered in the wearable electronic device, and may include, for example, at least one of a sweat component, a vein image, an electrical signal of a pulse, a skin depth image of the registered user, and the like. In other words, whether to execute the initial authentication setting mode of FIG. 6A may be determined in operation 701. Hereinafter, it is assumed that the wearable electronic device has set and stored the initial authentication information of the user.

In operation 703, the electronic device may measure a skin color of the user by using the wearable electronic device. The wearable electronic device may generate a skin color image of the user by using the image sensor and the light emitter included in the sensor unit of the wearable electronic device, and may transmit skin color information including the generated skin color image to the electronic device through short-range wireless communication. In operation 705, the wearable electronic device may determine whether the amount of light (light amount) received through a light-receiving unit of the light emitter operated to generate a skin color image is greater than or equal to a threshold light amount. Herein, the threshold light amount may be a light amount that is necessary to generate a vein image. Also, the wearable electronic device may measure a light amount received through the light-receiving unit of the light emitter, and transmit light amount information including the measured light amount to the electronic device through short-range wireless communication. When receiving the light amount information, the wearable electronic device may analyze the received light amount information, extract the measured light amount, and determine whether the extracted light amount is greater than or equal to a threshold light amount. The threshold light amount may be determined corresponding to the initial authentication information set through the process of FIG. 6A. Accordingly, the wearable electronic device may measure a vein image by using the threshold light amount included in the initial authentication information, thus reducing the power consumption of the wearable electronic device.

When the measured light amount is greater than or equal to the threshold light amount, the wearable electronic device may proceed to operation 709; and when the measured light amount is not greater than or equal to the threshold light amount, the wearable electronic device may proceed to operation 707.

In operation 707, the wearable electronic device may control the light emitter to control the light amount. Also, the wearable electronic device may receive a light amount control signal from the electronic device through short-range wireless communication. Herein, the light amount control signal may include information for requesting control of the intensity of light emitted from the light-emitting unit included in the light emitter of the wearable electronic device. When receiving the light amount control signal, the wearable electronic device may control the light intensity set in the light-emitting unit of the light emitter and operate the light-emitting unit. For example, in response to the light amount control signal, the wearable electronic device may control the set light intensity to be higher or lower than the current level.

In operation 709, the wearable electronic device may generate a vein image of the user. Also, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 711, the wearable electronic device may determine whether a vein image storage request is received from the user. When a vein image storage request is received from the user, the wearable electronic device may proceed to operation 713; and when a vein image storage request is not received from the user, the wearable electronic device may return to operation 701.

In operation 713, the wearable electronic device may generate authentication information including the vein image and the skin color information and store the generated authentication information.

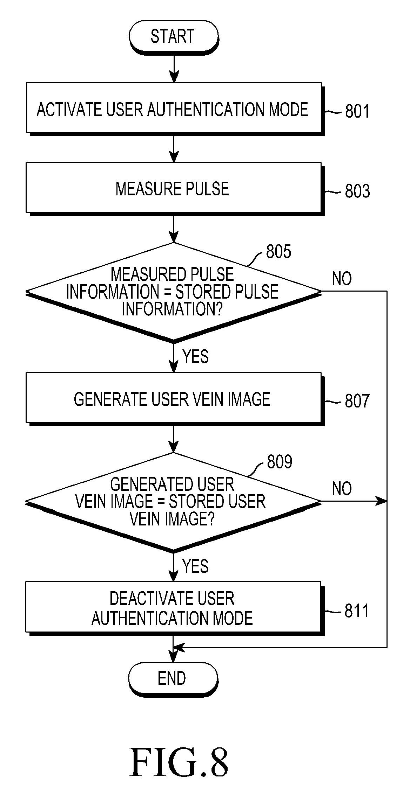

FIG. 8 is a flowchart illustrating a process of performing a user authentication operation in a wearable electronic device according to an embodiment of the present disclosure.

Referring to FIG. 8, in operation 801, the wearable electronic device may activate a user authentication mode. Herein, the user authentication mode is a mode for authenticating the user to determine whether the current user of the wearable electronic device has been registered in the wearable electronic device.

In operation 803, the wearable electronic device may measure a pulse of the user. For example, in operation 803, the wearable electronic device may measure a pulse of the user for a predtermined. Also, for example, the wearable electronic device may receive a pulse measurement request signal from the electronic device through short-range wireless communication. When receiving the pulse measurement request signal, the wearable electronic device may measure a pulse of the user by using the pulse measuring unit included in the sensor unit of the wearable electronic device, and transmit pulse information including a pulse measurement result to the electronic device through short-range wireless communication. Herein, the pulse information may include an electrical signal of the pulse of the user.

In operation 805, the wearable electronic device may compare the measured pulse information with the prestored pulse information included in the authentication information. When the measured pulse information is identical to the prestored pulse information, the wearable electronic device may proceed to operation 807; and when the measured pulse information is not identical to the prestored pulse information, the wearable electronic device may output a message indicating that the current user of the wearable electronic device is not a registered user, and continue to execute the user authentication mode.

In operation 807, the wearable electronic device may generate a vein image of the user. Also, for example, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 809, the wearable electronic device may compare the generated vein image with the prestored vein image included in the authentication information. When the generated vein image is identical to the prestored vein image, the wearable electronic device may proceed to operation 811; and when the generated vein image is not identical to the prestored vein image, the wearable electronic device may output a message indicating that the current user of the wearable electronic device is not a registered user, and continue to execute the user authentication mode.

In operation 811, the wearable electronic device may determine the current user of the wearable electronic device as a registered use, deactivate the user authentication mode, and display a standby screen of the wearable electronic device.

Although it has been described that a pulse of the user is used to authenticate the user in operations 803 and 805 of FIG. 8, any other biometric information may be used to authenticate the user. For example, the wearable electronic device may use a skin color of the user to authenticate the user. In various embodiments of the present disclosure, the wearable electronic device may also perform other operations in addition to an operation of authenticating the user by using a pulse of the user. For example, based on the measured pulse information, the wearable electronic device may determine that the user is in an emergency situation.

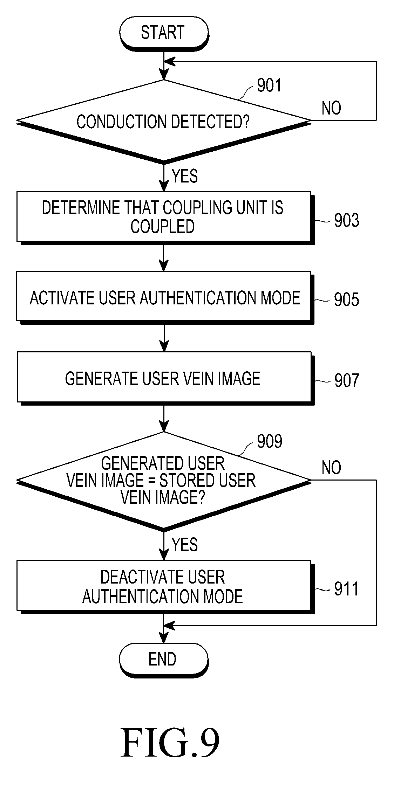

FIG. 9 is a flowchart illustrating a process of performing a user authentication operation in a wearable electronic device according to an embodiment of the present disclosure.

Referring to FIG. 9, in operation 901, the wearable electronic device may determine whether conduction is detected through the coupling unit of the wearable electronic device. For example, the wearable electronic device may have a coupling unit including a coupling protrusion (e.g., 405) and a coupling hook (e.g., 403) as illustrated in FIG. 4, and may use a conduction detecting unit included in the wearable electronic device to determine whether a current flows between the coupling protrusion and the coupling hook. When conduction is detected, the wearable electronic device may proceed to operation 903; and when conduction is not detected, the wearable electronic device may repeat operation 901. Also, for example, the wearable electronic device may transmit a conduction detection signal to the electronic device through short-range wireless communication.

In operation 903, the wearable electronic device may determine that the coupling unit is coupled. In operation 905, the wearable electronic device may activate a user authentication mode. Herein, the user authentication mode is a mode for authenticating the user, and refers to a mode for determining whether the current user of the wearable electronic device has been registered in the wearable electronic device.

In operation 907, the wearable electronic device may generate a vein image of the user. Also, for example, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 909, the wearable electronic device may compare the generated vein image with the prestored vein image included in the authentication information. When the generated vein image is identical to the prestored vein image, the wearable electronic device may proceed to operation 911; and when the generated vein image is not identical to the prestored vein image, the wearable electronic device may output a message indicating that the current user of the wearable electronic device is not a registered user. When the message is output, functions other than a security mode application of the wearable electronic device may be used.

Also, the user authentication mode may be repeatedly executed when the user wears the wearable electronic device or when the user enters a separate input.

In operation 911, the wearable electronic device may determine the current user of the wearable electronic device as a registered user and deactivate the user authentication mode.

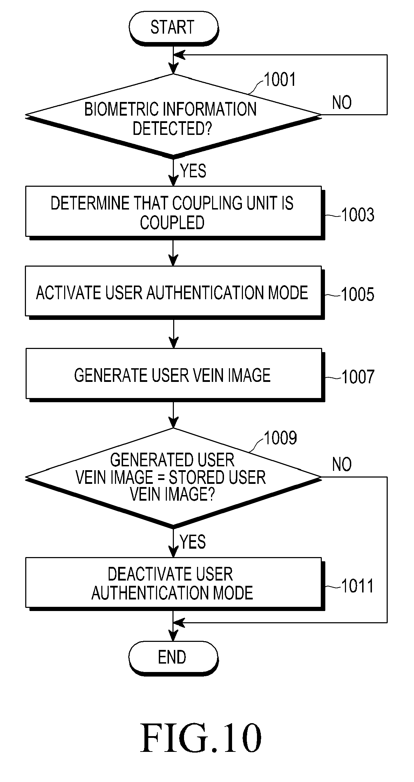

FIG. 10 is a flowchart illustrating a process of detecting a coupling of a wearable electronic device and performing a user authentication operation in a wearable electronic device according to an embodiment of the present disclosure.

Referring to FIG. 10, in operation 1001, the wearable electronic device may determine whether biometric information is detected through the sensor unit of the wearable electronic device. For example, the wearable electronic device may determine whether biometric information (e.g., a skin color, a pulse, a sweat component, etc. of the user) is detected through the sensor unit. Also, for example, when the biometric information is detected, the wearable electronic device may transmit a biometric information detection signal to the electronic device through short-range wireless communication.

When the biometric information is detected, the wearable electronic device may proceed to operation 1003; and when the biometric information is not detected, the wearable electronic device may repeat operation 1001.

In operation 1003, the wearable electronic device may determine that the coupling unit is coupled.

In operation 1005, the wearable electronic device may activate a user authentication mode. Herein, the user authentication mode is a mode for authenticating the user, and refers to a mode for determining whether the current user of the wearable electronic device has been registered in the wearable electronic device.

In operation 1007, the wearable electronic device may generate a vein image of the user. Also, for example, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 1009, the wearable electronic device may compare the generated vein image with the prestored vein image included in the authentication information. When the generated vein image is identical to the prestored vein image, the wearable electronic device may proceed to operation 1011; and when the generated vein image is not identical to the prestored vein image, the wearable electronic device may output a message indicating that the current user of the wearable electronic device is not a registered user. When the message is output, functions other than a security mode application of the wearable electronic device may be used.

Also, the user authentication mode may be repeatedly executed when the user wears the wearable electronic device or when the user enters a separate input.

In operation 1011, the electronic device may determine the current user of the wearable electronic device as a registered user and deactivate the user authentication mode.

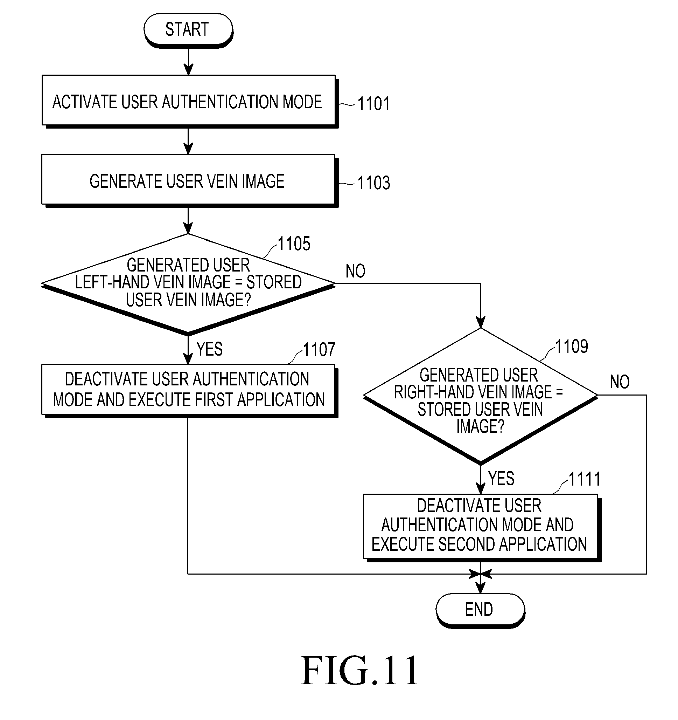

FIG. 11 is a flowchart illustrating a process of executing an application of a wearable electronic device according to a hand of the user to which the wearable electronic device is coupled according to an embodiment of the present disclosure.

Referring to FIG. 11, in operation 1101, the wearable electronic device may activate a user authentication mode. Herein, the user authentication mode is a mode for authenticating the user, and refers to a mode for determining whether the current user of the electronic device has been registered in the electronic device.

In operation 1103, the electronic device may generate a vein image of the user by using the wearable electronic device. Also, for example, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 1105, the wearable electronic device may determine whether the generated vein image is identical to the prestored left-hand vein image included in the authentication information. When the generated vein image is identical to the prestored left-hand vein image, the wearable electronic device may proceed to operation 1107; and when the generated vein image is not identical to the prestored left-hand vein image, the wearable electronic device may proceed to operation 1109.

In operation 1107, the wearable electronic device may determine the current user of the wearable electronic device as a registered use, deactivate the user authentication mode, and execute at least one first application. Herein, the first application refers to an application that is daily used and has an average security level, and may include, for example, a bus card application, a retail payment application, a message application or the like.

In operation 1109, the wearable electronic device may determine whether the generated vein image is identical to the prestored right-hand vein image included in the authentication information. When the generated vein image is identical to the prestored right-hand vein image, the wearable electronic device may proceed to operation 1111; and when the generated vein image is not identical to the prestored right-hand vein image, the wearable electronic device may output a message indicating that the current user of the wearable electronic device is not a registered user, and continue to execute the user authentication mode.

In operation 1111, the wearable electronic device may determine the current user of the wearable electronic device as a registered user, deactivate the user authentication mode, and execute at least one second application. Herein, the second application refers to an application that is non-daily used and has a security level higher than an average security level, and may include, for example, an electronic banking application or an identification application.

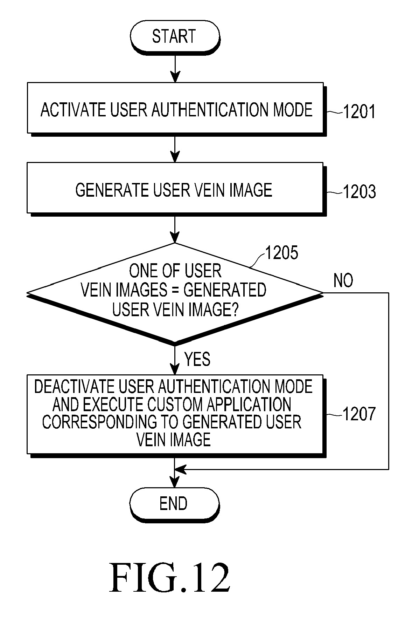

FIG. 12 is a flowchart illustrating a process of performing an application according to a user in a wearable electronic device according to an embodiment of the present disclosure.

Referring to FIG. 12, in operation 1201, the wearable electronic device may activate a user authentication mode. Herein, the user authentication mode is a mode for authenticating the user, and refers to a mode for determining whether the current user of the wearable electronic device has been registered in the wearable electronic device.

In operation 1203, the wearable electronic device may generate a vein image of the user. Also, for example, the wearable electronic device may receive a vein image generation request signal for requesting generation of a vein image of the user from the electronic device through short-range wireless communication. When receiving the vein image generation request signal, the wearable electronic device may generate a vein image of the user by using the image sensor and the light emitter included in the sensor unit of the wearable electronic device, and may transmit the generated vein image to the electronic device through short-range wireless communication.

In operation 1205, the wearable electronic device may determine whether the generated vein image is identical to one of prestored vein images of users included in the authentication information. When the generated vein image is identical to one of the prestored vein images, the wearable electronic device may proceed to operation 1207; and when the generated vein image is not identical to one of the prestored vein images, the wearable electronic device may output a message indicating that the current user of the wearable electronic device is not a registered user, and continue to execute the user authentication mode.

In operation 1207, the wearable electronic device may determine the current user of the wearable electronic device as a registered user, deactivate the user authentication mode, and execute a custom application corresponding to one of the prestored vein images. For example, the wearable electronic device may automatically connect with and synchronize with an e-mail server or a cloud server that is registered by the user corresponding to one of the prestored vein images.

As another example, when the user corresponding to one of the prestored vein images is a woman, the wearable electronic device may enter a woman mode to provide at least one application, which women execute more frequently than men, on a standby screen. As another example, when the user corresponding to one of the prestored vein images is a child, the wearable electronic device may enter a child mode to provide at least one application, which children execute more frequently than adults, on a standby screen, and does not provide at least one application that is forbidden to children. As another example, when the user corresponding to one of the prestored vein images is senior citizen, the wearable electronic device may enter senior citizen mode to provide at least one application, which senior citizens frequently execute, on a standby screen.

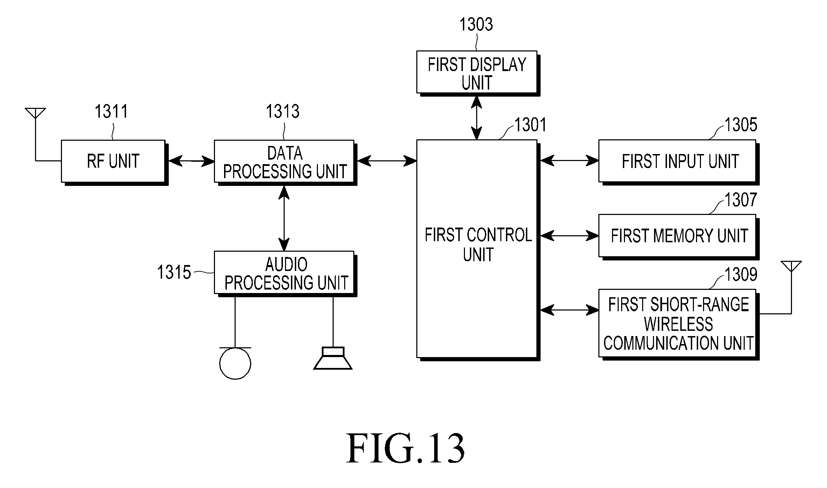

FIG. 13 is a block diagram of an electronic device according to an embodiment of the present disclosure.

Referring to FIG. 13, the electronic device may include a first control unit 1301, a first display unit 1303, a first input unit 1305, a first memory unit 1307, a first short-range wireless communication unit 1309, a Radio Frequency (RF) unit 1311, a data processing unit 1313, and an audio processing unit 1315.

The RF unit 1311 performs wireless communication with the wearable electronic device. In more detail, the RF unit 1311 includes an RF transmitter that up-converts and amplifies a transmission signal and an RF receiver that low-noise-amplifies and down-converts a received signal. The data processing unit 1313 includes a transmitter that encodes and modulates a transmission signal and a receiver that demodulates and decodes a received signal. Herein, the data processing unit 1313 may include a modem and a codec, and the codec may include a data codec that processes packet data and an audio codec that processes an audio signal such as voice.

The audio processing unit 1315 reproduces a received audio signal, which is output from the data processing unit 1313, through a speaker and transmits a transmission audio signal, which is generated from a microphone, to the data processing unit 1313. The first input unit 1305 includes keys for inputting numeral and text information and function keys for setting various functions. The first display unit 1303 displays a video signal and displays data that is requested from the first control unit 1301. When the first display unit 1303 is implemented as a capacitive or resistive touchscreen, the first input unit 1305 may include a minimum number of keys, and the first display unit 1303 may replace the first input unit 1305 to perform a key input function.

The first memory unit 1307 may include a program memory and a data memory. Herein, the program memory stores booting and Operating System (OS) programs for controlling general operations of the electronic device, and the data memory stores various data that is generated during the operation of the electronic device. For example, the first memory unit 1307 may store authentication information that is generated in an authentication setting mode. Herein, the authentication information may include biometric information of the user registered in the wearable electronic device. The wearable electronic device may transmit the biometric information to the electronic device, and the electronic device may store the received biometric information. The biometric information may include a sweat component, a vein image, an electrical signal of a pulse, a skin depth image, etc. of the user registered in the wearable electronic device. As another example, the first memory unit 1307 of the electronic device may store a left-hand vein image and a right-hand vein image of the user that are received from the wearable electronic device. As another example, the first memory unit 1307 of the electronic device may store authentication information of users that is received from the wearable electronic device.

The first short-range wireless communication unit 1309 performs a short-range wireless communication function of the electronic device. For example, short-range wireless communication may be Bluetooth, WiFi, or the like.

The first control unit 1301 of the electronic device may control overall operations of the wearable electronic device. For example, the first control unit 1301 may control the wearable electronic device to register authentication information in an authentication setting mode.

In an embodiment, the first control unit 1301 of the electronic device may determine whether an authentication setting mode activation request is received from the user of the wearable electronic device. Herein, the authentication setting mode refers to a mode for setting unique authentication information of the user to authenticate the user.

When the authentication setting mode activation request is received, the first control unit 1301 of the electronic device may control the wearable electronic device to measure a pulse of the user. For example, the first control unit 1301 may transmit a pulse measurement signal to the wearable electronic device through short-range wireless communication. Also, the first control unit 1301 may receive pulse information from the wearable electronic device through short-range wireless communication. Herein, the pulse information may include an electrical signal of the pulse of the user. The first control unit 1301 of the electronic device may determine whether a pulse measurement result of the wearable electronic device is within a threshold range. To this end, for example, the first control unit 1301 may detect a result converging to a predetermined cycle and pattern from a heart rate, which is measured by the wearable electronic device, and determine whether the result matches with the vein image.

Also, the first control unit 1301 of the electronic device may determine whether a pulse signal measured by the wearable electronic device is within a threshold amplitude or cycle.

When the pulse measurement result is within the threshold range, for example, when the pulse measurement result converges into the threshold range and is determined as the authentication information of the user of the wearable electronic device, the first control unit 1301 of the electronic device may control the wearable electronic device to generate a vein image of the user. For example, the first control unit 1301 of the electronic device may transmit a vein image generation request signal for requesting generation of a vein image of the user to the wearable electronic device through short-range wireless communication. Also, the first control unit 1301 of the electronic device may receive a vein image of the user from the wearable electronic device through short-range wireless communication.

The first control unit 1301 of the electronic device may determine whether a vein image storage request is received from the user of the wearable electronic device. When the vein image storage request is received, the first control unit 1301 of the electronic device may control the wearable electronic device to generate authentication information including the vein image and the pulse information and store the generated authentication information.

In another embodiment, the first control unit 1301 of the electronic device may control the wearable electronic device to determine whether an authentication setting mode activation request is received from the user of the wearable electronic device. When the authentication setting mode activation request is received, the first control unit 1301 of the electronic device may control the wearable electronic device to measure a skin color of the user. For example, the first control unit 1301 of the electronic device may receive a skin color signal measured by the wearable electronic device through short-range wireless communication. Also, the first control unit 1301 of the electronic device may receive skin color information of the user from the wearable electronic device through short-range wireless communication.

The first control unit 1301 may determine whether the amount of light (light amount) received through the light-receiving unit of the light emitter of the wearable electronic device operated to generate skin color information is greater than or equal to a threshold light amount. To this end, for example, the wearable electronic device may store light amount information, which has been used for initial authentication vein recognition, corresponding to the vein image and provide the light amount setting value as a default value in an authentication mode.

Also, for example, the threshold light amount may include a light amount that is necessary to generate a vein image. The wearable electronic device may analyze light amount information to extract a light amount, and the first control unit 1301 of the electronic device may receive information indicating whether the extracted light amount is greater than or equal to a threshold light amount through short-range wireless communication. Herein, the light amount information may include a measurement value of the amount of light received by the light-receiving unit of the light emitter of the wearable electronic device. When the measured light amount is smaller than the threshold light amount, the first control unit 1301 may control the light emitter of the wearable electronic device to control the light amount. For example, the first control unit 1301 may transmit a light amount control signal to the wearable electronic device through short-range wireless communication. Based on the light amount control signal, the wearable electronic device may control the light intensity set in the light-emitting unit of the light emitter and operate the light-emitting unit. For example, in response to the light amount control signal, the wearable electronic device may control the set light intensity to be higher or lower than the current level.

When the measured light amount is greater than or equal to the threshold light amount, the first control unit 1301 may control the wearable electronic device to generate a vein image of the user. For example, the first control unit 1301 of the electronic device may transmit a vein image generation request signal for requesting generation of a vein image of the user of the wearable electronic device to the wearable electronic device through short-range wireless communication. The first control unit 1301 may receive the vein image generated by the wearable electronic device through short-range wireless communication.

The first control unit 1301 of the electronic device may determine whether a vein image storage request is received from the user of the wearable electronic device. When the vein image storage request is received, the first control unit 1301 may control the wearable electronic device to generate authentication information including the vein image and the skin color information and store the generated authentication information. As another example, the first control unit 1301 of the electronic device may control the wearable electronic device to perform a user authentication operation based on the biometric information of the user of the wearable electronic device.

The first control unit 1301 of the electronic device may control the wearable electronic device to execute a user authentication mode. Herein, the user authentication mode is a mode for authenticating the user of the wearable electronic device, and refers to a mode for determining whether the user has been registered in the wearable electronic device.

The first control unit 1301 may control the wearable electronic device to measure a pulse of the user. For example, the first control unit 1301 may receive a pulse signal measured by the wearable electronic device through short-range wireless communication.

The first control unit 1301 of the electronic device may control the wearable electronic device to compare the measured pulse information with the prestored pulse information included in the authentication information. When the measured pulse information is not identical to the prestored pulse information, the first control unit 1301 of the electronic device may control the wearable electronic device to output a message indicating that the current user is not a registered user. Also, the message may be output from the electronic device.

When the measured pulse information is identical to the prestored pulse information, the first control unit 1301 of the electronic device may control the wearable electronic device to generate a vein image of the user. For example, the first control unit 1301 may transmit a vein image generation request signal for requesting generation of a vein image of the user of the wearable electronic device to the wearable electronic device through short-range wireless communication. The first control unit 1301 may receive the vein image of the user from the wearable electronic device through short-range wireless communication.

The first control unit 1301 of the electronic device may control the wearable electronic device to compare the generated vein image with the prestored vein image included in the authentication information. When the generated vein image is not identical to the prestored vein image, the first control unit 1301 of the electronic device may control the wearable electronic device to output a message indicating that the current user is not a registered user. When the generated vein image is identical to the prestored vein image, the first control unit 1301 of the electronic device may control the wearable electronic device to determine the current user of the wearable electronic device as a registered user, deactivate the user authentication mode, and display a standby screen.

As another example, the first control unit 1301 of the electronic device may control the wearable electronic device to perform a user authentication operation, when detecting a coupling of the wearable electronic device.

In an embodiment, the first control unit 1301 of the electronic device may control the wearable electronic device to determine whether conduction is detected through the coupling unit of the wearable electronic device. For example, the wearable electronic device may have a coupling unit including a coupling protrusion (e.g., 405) and a coupling hook (e.g., 403) as illustrated in FIG. 4, and the first control unit 1301 of the electronic device may control the wearable electronic device to determine whether a current flows between the coupling protrusion and the coupling hook, by using a conduction detecting unit included in the wearable electronic device. Accordingly, when the conduction is detected, the first control unit 1301 of the electronic device may control the wearable electronic device to transmit a conduction detection signal to the electronic device through short-range wireless communication.

When determining that the coupling unit of the wearable electronic device is coupled, the first control unit 1301 of the electronic device may control the wearable electronic device to execute a user authentication mode. Herein, the user authentication mode is a mode for authenticating the user, and refers to a mode for determining whether the current user of the wearable electronic device has been registered in the wearable electronic device.

The first control unit 1301 of the electronic device may control the wearable electronic device to generate a vein image of the user. For example, the first control unit 1301 of the electronic device may control the wearable electronic device to receive a vein image generation request signal from the first control unit 1301 of the electronic device, generate a vein image of the user by using the image sensor and the light emitter included in the sensor unit, and transmit the generated vein image to the electronic device through short-range wireless communication.

The first control unit 1301 of the electronic device may control the wearable electronic device to compare the generated vein image with the prestored vein image included in the authentication information. When the generated vein image is not identical to the prestored vein image, the first control unit 1301 of the electronic device may control the wearable electronic device to output a message indicating that the current user of the wearable electronic device is not a registered user. When the message is output, the first control unit 1301 of the electronic device may perform control such that functions other than a security mode application of the wearable electronic device may be used. Also, the first control unit 1301 of the electronic device may perform control such that the user authentication mode may be repeatedly executed when the user wears the wearable electronic device or when the user enters a separate input.

When the generated vein image is identical to the prestored vein image, the first control unit 1301 of the electronic device may control the wearable electronic device to determine the current user of the wearable electronic device as a registered user and deactivate the user authentication mode.

In another embodiment, the first control unit 1301 of the electronic device may control the wearable electronic device to execute a user authentication mode. The first control unit 1301 of the electronic device may control the wearable electronic device to determine whether the biometric information is detected through the sensor unit of the wearable electronic device. For example, the wearable electronic device may determine whether biometric information (e.g., a skin color, a pulse, a sweat component, etc. of the user) is detected through the sensor unit. When the biometric information is detected, the wearable electronic device may transmit a biometric information detection signal to the electronic device through short-range wireless communication. The wearable electronic device may determine the detection of the biometric information based on the biometric information detection signal and transmit the detected information to the electronic device.

When the wearable electronic device detects the biometric information, the first control unit 1301 of the electronic device may determine that the coupling unit of the wearable electronic device is coupled. The first control unit 1301 may control the wearable electronic device to generate a vein image of the user. The first control unit 1301 may control the wearable electronic device to compare the generated vein image with the prestored vein image included in the authentication information. When the generated vein image is not identical to the prestored vein image, the first control unit 1301 may output a message indicating that the current user is not a registered user. When the generated vein image is identical to the prestored vein image, the first control unit 1301 may control the wearable electronic device to determine the current user of the wearable electronic device as a registered user, deactivate the user authentication mode, and display a standby screen.

As another example, the first control unit 1301 of the electronic device may control the wearable electronic device to execute different applications depending on the hands of the user to which the wearable electronic device is coupled.

In more detail, the first control unit 1301 may execute a user authentication mode. The first control unit 1301 may control the wearable electronic device to generate a vein image of the user.

The first control unit 1301 may determine whether the vein image generated by the wearable electronic device is identical to the prestored left-hand vein image included in the authentication information. When the generated vein image is identical to the prestored left-hand vein image, the first control unit 1301 may control the wearable electronic device to determine the current user of the wearable electronic device as a registered user, deactivate the user authentication mode, and execute at least one first application. Herein, the first application refers to an application that is daily used and has an average security level, and may include, for example, a bus card application, a retail payment application, a message application, or the like.

When the generated vein image is not identical to the prestored left-hand vein image, the first control unit 1301 may control the wearable electronic device to determine whether the generated vein image is identical to the prestored right-hand vein image included in the authentication information. When the generated vein image is not identical to the prestored right-hand vein image, the wearable electronic device may control the wearable electronic device to output a message indicating that the current user is not a registered user, and continue to execute the user authentication mode.

When the generated vein image is identical to the prestored right-hand vein image, the first control unit 1301 may control the wearable electronic device to register the user, deactivate the user authentication mode, and execute at least one second application. Herein, the second application refers to an application that is non-daily used and has a security level higher than an average security level, and may include, for example, an electronic banking application or an identification application.

As another example, the first control unit 1301 of the electronic device may control the wearable electronic device to execute different applications depending on the users using the wearable electronic device.

In more detail, the first control unit 1301 may control the wearable electronic device to execute a user authentication mode. The first control unit 1301 may control the wearable electronic device to generate a vein image of the user. The first control unit 1301 may control the wearable electronic device to determine whether the vein image generated by the wearable electronic device is identical to one of the prestored vein images of the users included in the authentication information. When the generated vein image is not identical to one of the prestored vein images, the first control unit 1301 may output a message indicating that the current user is not a registered user.

When the generated vein image is identical to one of the prestored vein images, the first control unit 1301 may control the wearable electronic device to determine the current user of the wearable electronic device as a registered user, deactivate the user authentication mode, and execute a custom application corresponding to one of the prestored vein images. For example, the first control unit 1301 may control the wearable electronic device to automatically connect with and synchronize with an e-mail server or a cloud server that is registered by the user corresponding to one of the prestored vein images.

As another example, when the user corresponding to one of the prestored vein images is a woman, the first control unit 1301 may control the wearable electronic device to enter a woman mode to provide at least one application, which women execute more frequently than men, on a standby screen. As another example, when the user corresponding to one of the prestored vein images is a child, the first control unit 1301 may control the wearable electronic device to enter a child mode to provide at least one application, which children execute more frequently than adults, on a standby screen. Also, the first control unit 1301 may control the wearable electronic device not to provide at least one application that is forbidden to children. As another example, when the user corresponding to one of the prestored vein images is a senior citizen, the first control unit 1301 may control the wearable electronic device to enter a senior citizen mode to provide at least one application, which senior citizens frequently execute, on a standby screen.

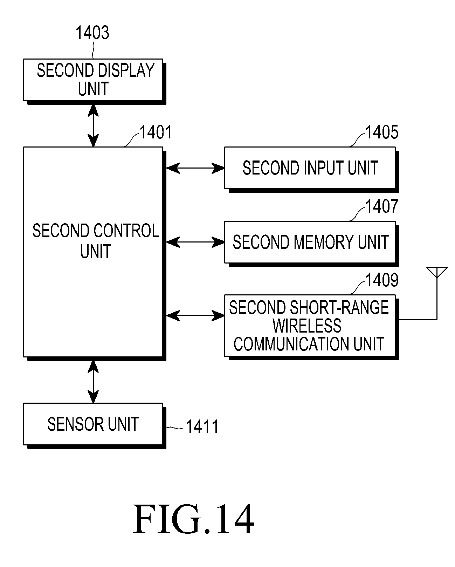

FIG. 14 is a block diagram of a wearable electronic device according to an embodiment of the present disclosure.

Referring to FIG. 14, the wearable electronic device may include a second control unit 1401, a second display unit 1403, a second input unit 1405, a second memory unit 1407, a second short-range wireless communication unit 1409, and a sensor unit 1411.

The second input unit 1405 includes keys for inputting numeral and text information and function keys for setting various functions. The second display unit 1403 displays a video signal, and displays data that is requested from the second control unit 1401. When the second display unit 1403 is implemented as a capacitive or resistive touchscreen, the second input unit 1405 may include a minimum number of keys, and the second display unit 1403 may replace the second input unit 1405 to perform a key input function.

The second memory unit 1407 may include a program memory and a data memory. Herein, the program memory stores booting and OS programs for controlling general operations of the wearable electronic device, and the data memory stores various data that is generated during the operation of the wearable electronic device. The second short-range wireless communication unit 1409 performs a short-range wireless communication function of the wearable electronic device. For example, short-range wireless communication may be Bluetooth, WiFi, or the like.

The sensor unit 1411 may include a light emitter and an image sensor. Herein, the light emitter may include a light-emitting unit and a light-receiving unit, and the image sensor may generate a vein image of the wrist or the back of the hand of the user or a skin color image of the user. The sensor unit 1411 may include a pulse measuring unit that measures a pulse of the user and converts the measured pulse into an electrical signal. The sensor unit 1411 may include a conduction detecting unit. As illustrated in FIG. 4, the wearable electronic device may be mounted with a coupling unit including a coupling protrusion (e.g., 405) and a coupling hook (e.g., 403). For example, the coupling protrusion may include a conductor, and the coupling hook may include a conductor and a nonconductor. A portion of the coupling hook coupling with and contacting with the coupling protrusion may be a conductor. Accordingly, a voltage may be applied to the coupling unit, and the conduction detecting unit may detect that a current flows between the coupling protrusion and the coupling hook. The sensor unit 1411 may include a sweat component analyzing unit for analyzing a sweat component of the user.

The second control unit 1401 controls overall functions of the wearable electronic device. For example, when receiving a pulse measurement request signal through short-range wireless communication, the second control unit 1401 may measure a pulse of the user by using the pulse measuring unit included in the sensor unit 1411, and transmit pulse information including a pulse measurement result to the electronic device through short-range wireless communication.

As another example, when receiving a vein image generation request signal through short-range wireless communication, the second control unit 1401 may generate a vein image of the user by using the image sensor and the light emitter included in the sensor unit 1411, and may transmit the generated vein image to the electronic device through short-range wireless communication. As another example, when detecting the conduction between the coupling hook and the coupling protrusion through the conduction detecting unit included in the sensor unit 1411, the second control unit 1401 may transmit a conduction detection signal to the electronic device through short-range wireless communication. As another example, when detecting the biometric information through the sensor unit 1411, the second control unit 1401 may transmit a biometric information detection signal to the electronic device through short-range wireless communication.

Also, the second control unit 1401 of the wearable electronic device may directly perform the operation of the first control unit 1301 of the electronic device for controlling the wearable electronic device, which has been described with reference to FIG. 13.

As is apparent from the foregoing description, according to the various embodiments of the present disclosure, the user is authenticated in the electronic device based on the unique biometric information of the user, thereby maximizing the security of the electronic device. Also, according to the various embodiments of the present disclosure, the unique biometric information of the user is extracted by using the external device that is capable of communicating with the electronic device, thereby maximizing the security of the electronic device.

An apparatus and method for authenticating the user in the electronic device according to the various embodiments of the present disclosure may also be embodied as computer-readable codes on a computer-readable recording medium. The computer-readable recording medium may be any data storage device that may store data which may be thereafter read by a computer system. Examples of the computer-readable recording medium include Read Only Memories (ROMs), Random Access Memories (RAMs), optical disks, magnetic tapes, floppy disks, hard disks, nonvolatile memories, and carrier waves (such as data transmission through the Internet). The computer-readable recording medium may also be distributed over network-coupled computer systems so that the computer-readable code may be stored and executed in a distributed fashion.

While the present disclosure has been shown and described with reference to various embodiments thereof, it will be understood by those skilled in the art that various changes in form and details may be made therein without departing from the spirit and scope of the present disclosure as defined by the appended claims and their equivalents.

## Claims

1. An electronic device for controlling a wearable electronic device to authenticate a user, the electronic device comprising:
a short-range wireless communication unit configured to receive biometric information of the user from the wearable electronic device; and
a control unit configured to determine whether the user has been registered in the wearable electronic device, based on the biometric information and stored authentication information, and control the wearable electronic device to authenticate the user according to a result of the determination.

2. The electronic device of claim 1, wherein the biometric information comprises at least one of a sweat component, a vein image, an electrical signal of a pulse, and a skin color image of the user.

3. The electronic device of claim 1, wherein, when an electrical signal of a pulse of the user included in the biometric information is identical to an electrical signal of a pulse of the registered user included in the authentication information, the control unit transmits a vein image generation request signal for requesting generation of a vein image of the user to the wearable electronic device through the short-range wireless communication unit.

4. The electronic device of claim 1, wherein the control unit determines whether the wearable electronic device is coupled, and transmits a signal for requesting generation of the biometric information to the wearable electronic device when the wearable electronic device is coupled.

5. The electronic device of claim 4, wherein, when receiving a conduction detection signal, which indicates conduction between a coupling protrusion and a coupling hook included in the wearable electronic device, or a biometric information detection signal, which indicates detection of the biometric information, from the wearable electronic device, the control unit determines that the wearable electronic device is coupled.

6. A wearable electronic device for authenticating a user, the wearable electronic device comprising:
an image sensor and a light emitter mounted on one surface of the wearable electronic device; and
a lens or a waveguide comprising at least one opening on a rear surface thereof.

7. The wearable electronic device of claim 6, further comprising a pulse measuring unit configured to measure a pulse of the user and convert the measured pulse into an electrical signal.

8. The wearable electronic device of claim 7, further comprising a control unit configured to generate a vein image of the user, to which the wearable electronic device is coupled, by using the image sensor and the light emitter, and generate pulse information of the user by using the pulse measuring unit.

9. The wearable electronic device of claim 6, further comprising:
a coupling hook to which a first reference voltage is applied;
a coupling protrusion to which a second reference voltage is applied; and
a conduction detecting unit configured to detect conduction between the coupling hook and the coupling protrusion.

10. The wearable electronic device of claim 9, further comprising a control unit configured to detect the conduction by using the conduction detecting unit, and generate a biometric information detection signal indicating detection of biometric information.

11. A method for authenticating a user by a wearable electronic device, the method comprising:
generating biometric information of the user;
generating pulse information of the user; and
generating a vein image of the user.

12. The method of claim 11, wherein the vein image is generated by using an image sensor and a light emitter.

13. The method of claim 11, further comprising:
detecting conduction between a coupling hook and a coupling protrusion by using a conduction detecting unit; and
generating a biometric information detection signal indicating detection of the biometric information.

14. The method of claim 12, wherein the image sensor and the light emitter are mounted on an inner surface of the wearable electronic device to face each other.

15. The method of claim 11, wherein the coupling protrusion comprises a conductor, and the coupling hook comprises a conductor and a nonconductor, so that a voltage is applied to the wearable electronic device when the coupling protrusion and the coupling hook are coupled with each other.
